Europäisches Patentamt

European Patent Office

Office européen des brevets

(11) Numéro de publication: **0 210 907 B1**

(12) **FASCICULE DE BREVET EUROPEEN**

(45) Date de publication du fascicule du brevet:
13.09.89

(21) Numéro de dépôt: **86401520.1**

(22) Date de dépôt: **09.07.86**

(51) Int. Cl.⁴: **C07C 69/54,** C07C 67/03

(54) Procédé de préparation d'esters méthacryliques par transestérification.

(30) Priorité: **11.07.85 FR 8510674**

(43) Date de publication de la demande:
**04.02.87 Bulletin 87/6**

(45) Mention de la délivrance du brevet:
**13.09.89 Bulletin 89/37**

(84) Etats contractants désignés:
**AT BE CH DE FR GB IT LI LU NL SE**

(56) Documents cités:
**DE-A- 2 805 702**
**GB-A- 2 005 252**

(73) Titulaire: **SOCIETE FRANCAISE D'ORGANO-SYNTHESE,
15, boulevard de l'Amiral Bruix, F-75116 Paris(FR)**

(72) Inventeur: **Gabillet, Philippe, 33bis, rue Daudeville,
F-75018 Paris(FR)**

(74) Mandataire: **Fabre, Madeleine-France et al,
RHONE-POULENC INTERSERVICES Service Brevets
Chimie 25, quai Paul Doumer, F-92408 Courbevoie
Cédex(FR)**

## Description

La présente invention concerne un procédé de préparation d'esters méthacryliques par transestérification.

On a décrit, dans le brevet français 2 405 237, notamment un procédé de préparation d'esters méthacryliques par transestérification ; il a été signalé dans ledit brevet que le procédé était réalisé en présence d'un catalyseur de transestérification qui est de l'hydroxyde de lithium utilisé à raison de 0,01 à 2 % en poids par rapport à l'ensemble des réactifs et en présence d'un inhibiteur - connu - de polymérisation du méthacrylate ; ledit brevet mentionne également que le mélange réactionnel est chauffé, à reflux total, jusqu'au moment où en tête de colonne on atteint la température de l'azéotrope méthacrylate de méthyle/ méthanol, cet azéotrope permettant l'évacuation progressive du méthanol présent dans le milieu réactionnel.

L'étude systématique des réactions de transestérification du méthacrylate de méthyle avec les alcools supérieurs en utilisant un composé de lithium (hydroxyde de préférence ou carbonate) comme catalyseur a montré qu'il était possible, et industriellement souhaitable, de n'utiliser que des quantités de catalyseur inférieures à celles préconisées dans le brevet 2 405 237 à condition, d'une part, d'introduire le catalyseur progressivement dans le milieu réactionnel et, d'autre part, d'opérer en présence d'un corps azéotropeur inerte qui donne naissance, avec le méthanol présent dans le milieu, à un azéotrope bouillant à une température inférieure à environ 60° C (à pression atmosphérique).

La présente invention concerne donc un procédé de transestérification du méthacrylate de méthyle par un alcool lourd en présence de lithium comme catalyseur, caractérisé en ce que la quantité de lithium utilisée est comprise entre 6 et 30 ppm (exprimée en lithium) d'hydroxyde ou de carbonate de lithium, que le composé de lithium utilisé est ajouté progressivement au milieu réactionnel et que la réaction est effectuée en présence d'un azéotropeur donnant, avec le méthanol, un azéotrope bouillant à température inférieure à environ 60° C.

La quantité de catalyseur utilisée est exprimée par rapport aux réactifs à savoir la somme du méthacrylate de méthyle et de l'alcool.

Bien évidemment, la réaction s'effectuera, comme connu, en présence d'un inhibiteur de polymérisation des méthacrylates. Comme inhibiteurs de polymérisation, on peut utiliser par exemple, de façon non limitative : monométhyl éther de l'hydroquinone, hydroquinone, 2,6-dit-Bu de paracrésol, 3,5-dit-butyl hydroxy-4 anisole, 2,5-dit-Bu hydroxy-4 anisole, N,N'-dinaphtyl paraphénylène diamine, 2-t-Bu 4-méthoxy phénol, ainsi que tout autre inhibiteur de polymérisation radicalaire, les quantités d'inhibiteurs variant de 100 à 1 400 ppm (pondéralement sur la quantité théorique d'ester attendu).

Les alcools utilisables pour réaliser la transestérification du méthacrylate de méthyle selon l'invention sont très nombreux. On peut citer de façon non limitative : les monoalcools aliphatiques linéaires ou ramifiés, tels que le n-butanol, le n-propanol, l'alcool laurique, l'alcool stéarique, le 2-éthyl hexanol ; les alcools cycloaliphatiques, tels que le cyclohexanol ; les alcools aromatiques comme l'alcool benzylique ; les alcools portant d'autres groupes fonctionnels, l'éther monométhylique de l'éthylène glycol, l'éther monoisopropylique de l'éthylène glycol ; les alcools insaturés, tels que l'alcool allylique, les aliphatiques tels que le monoéthylène glycol, le diéthylène glycol, le butanediol, le triméthylol propane, les alcools transestérifiants pouvant être d'origine naturelle ou synthétique.

Le catalyseur utilisé est le lithium sous forme de lithine (calcinée ou monohydratée) ou de carbonate de lithium.

Comme azéotropeur, on pourra utiliser tout produit inerte (par exemple et de préférence un hydrocarbure saturé comme l'hexane) donnant, avec le méthanol, un azéotrope bouillant à une température inférieure à 60° C. En fait, l'emploi de cet azéotropeur permet l'élimination du méthanol présent (ou formé) dans le milieu réactionnel mais en évitant la formation de l'azéotrope méthacrylate de méthyle-méthanol qui s'évacue à 65° C environ.

Selon l'invention, le catalyseur doit être introduit progressivement dans le milieu réactionnel. En fait, il faut éviter, en maintenant une concentration du catalyseur à un très bas niveau, qu'il y ait consommation dudit catalyseur par réaction du lithium avec un des réactifs présents. Le délai pendant lequel le catalyseur doit être introduit dans le milieu réactionnel varie avec les conditions expérimentales et la cinétique de la réaction ; on admettra comme règle que le catalyseur doit être introduit dans le milieu réactionnel pendant une période qui s'étend entre 1/10 et 1/2 de la durée totale de la réaction.

Il a été trouvé également, et cela constitue un aspect complémentaire de l'invention, que l'activité catalytique du lithium peut être renforcée par l'utilisation d'éthers couronnes et/ou de cryptants. Parmi ces cocatalyseurs utilisables on peut citer : le tétraoxo-1,4,7,10 cyclododécane (crown 2-4)- ou le tris (dioxa-3,6 heptyl) amine (TDA1) ; les quantités à utiliser, exprimées en moles par mole d'ion Li+, sont les suivantes : 0,01 à 2 moles par mole.

La transestérification est effectuée de la façon suivante : on charge l'alcool transestérifiant, l'inhibiteur de polymérisation et un solvant azéotropeur et on chauffe le temps nécessaire pour déshydrater le milieu.

Lorsque le milieu est suffisamment déshydraté, que la température masse se situe entre 100 et 140° C et après neutralisation des acidités résiduelles des réactifs, on commence simultanément l'introduction du méthacrylate de méthyle et d'une solution méthanolique d'hydroxyde de lithium ou d'une suspension méthanolique de carbonate de lithium. Le méthanol formé est extrait du milieu réactionnel par distillation azéotropique à pression atmosphérique.

Un barbotage d'air peut être effectué afin de renforcer l'efficacité de l'inhibiteur de polymérisation. De même, l'ajout de cryptants ou d'éthers couronnes renforce l'effet catalytique du catalyseur utilisé.

Les produits obtenus selon ce procédé peuvent être facilement isolés de la façon suivante :

- après une distillation sous pression réduite, afin d'éliminer l'hexane et l'excès de composés légers, on peut soit lorsque l'ester obtenu présente une volatilité suffisante le purifier par distillation, soit dans le cas contraire lui appliquer un traitement spécifique dépendant étroitement du type d'inhibiteur utilisé.

Traitement 1

Ce traitement est adapté aux inhibiteurs de polymérisation radicalaire contenant un groupement azoté. Ce traitement consiste en un passage sur terre ou noir acide suivi d'une filtration, ce passage sur terre acide pouvant être éventuellement précédé d'une attaque acide.

Traitement 2

Ce traitement particulièrement adapté aux inhibiteurs de polymérisation radicalaire de types quinoniques et phénoliques consiste en un passage sur terre ou noir basique suivi d'une filtration, ce passage pouvant être avantageusement précédé d'une attaque basique.

Les exemples non limitatifs suivants illustrent l'invention.

Exemple 1

Dans un ballon de six litres à sept tubulures muni d'un thermomètre, d'une agitation, d'une ampoule d'introduction d'hexane, d'une ampoule d'introduction de catalyseur, d'une ampoule d'introduction de méthacrylate de méthyle, d'un barbotage d'air et surmonté d'une colonne à distiller (Ø int. : 40 mm, H : 1,50 m, 18 plateaux réels) d'une tête de colonne suivie d'un dispositif de condensation et d'un décanteur horizontal, maintenu à 10° C, contenant une prise de température, un soutirage de couche inférieure et un recyclage de couche supérieure en tête de colonne, on introduit : 1573 g d'alcool stéarique (6 moles), 600 ml d'hexane et 1 g de N-N'-dinaphtyl paraphénylène diamine, 5,36 ml d'une solution de 2,49 % d'hydroxyde de lithium calciné dans le méthanol, après 45 min de séchage et, lorsque la colonne est en équilibre, on commence simultanément les coulées de méthacrylate de méthyle (690,9 g en 0 h 45) et de solution méthanolique de lithine (10,24 ml en 1 h 30).

La température masse est maintenue entre 115° C et 120° C par addition éventuelle d'hexane.

L'hétéroazéotrope est refroidi à 10° C afin de pouvoir décanter la couche inférieure. Après 4 h de réaction, on obtient un taux de conversion sur l'alcool de 98 %.

Exemple 2

Même mode opératoire qu'à l'exemple 1, mais l'alcool transestérifiant est une coupe isodécanolique (PM : 160,3 g moyen). On obtient une conversion de 100 % après 4 h de réaction.

Exemple 3

Dans un réacteur inox de 300 l équipé de façon similaire au ballon de 6 l décrit dans l'exemple 1, on charge : 80 kg d'alcool stéarique (PM moyen : 262,15 g), 25 kg d'hexane et 50 g de N,N'-dinaphtyl paraphénylène diamine et 5,41 g de monohydrate d'hydroxyde de lithium.

Puis après déshydratation du milieu réactionnel en 0 h 15, on commence simultanément l'introduction de 36,6 kg de méthacrylate de méthyle (durée : 0 h 35) et de 19,0 g de monohydrate d'hydroxyde de lithium en solution dans 1,2 kg de méthanol (durée : 1 h 30). Après 5 h de réaction, on rajoute 5 g de monohydrate d'hydroxyde de lithium en solution dans 200 g de méthanol. Après 6 h de réaction, le taux de conversion de l'alcool stéarique est de 98,2 % (déterminé par chromatographie).

Exemple 4

Même mode opératoire qu'à l'exemple 1, mais 0.021 g de TDA1 de Rhône-Poulenc sont mélangés au méthacrylate de méthyle à introduire.

On obtient après 4 h de réaction une conversion de l'alcool de 99,2 %.

Exemple 5

Même mode opératoire qu'à l'exemple 1, mais l'alcool transestérifiant est une coupe laurique de poids moléculaire moyen 207 g et l'inhibiteur de polymérisation est le 2,6-dit-Bu paracrésol. On obtient après 4 h de réaction une conversion de 98,5 %.

Exemple 6

Même mode opératoire qu'à l'exemple 1, mais l'alcool transestérifiant est une coupe laurique de poids moléculaire moyen 207 g et l'inhibiteur de polymérisation est le 2-tBu-4 méthoxy-phénol. On obtient après 4 h de réaction une conversion de 98,7 %.

Exemple 7

500 g de méthacrylate de stéaryle brut obtenu suivant le mode opératoire de l'exemple 1 et contenant 500 ppm de N,N'-dinaphtyl paraphénylène diamine sont placés dans un ballon pentacol de 1 l ; ce ballon muni d'une agitation de type demi-lune est surmonté d'un réfrigérant direct, d'un thermomètre, d'un plongeant permettant un bullage d'air, et d'un dispositif d'introduction de solide. La température masse est portée à 80-90° C sous agitation et bullage d'air, puis on introduit rapidement 6 g de Fulcat 22 B. La température est alors maintenue à 80-90° C durant 0 h 30, puis le milieu réactionnel est refroidi à 50° C et filtré sur une précouche de clarcel DIC. On obtient 492 g de méthacrylate de stéaryle contenant 75 ppm de N,N'-dinaphtyl paraphénylène diamine. Le gâteau de filtration peut être avantageusement rincé avec de l'hexane afin de recycler l'inhibiteur de polymérisation et le méthacrylate de stéaryle retenu.

## Exemple 8

On utilise le même mode opératoire que celui décrit dans l'exemple 6 avec 1242 g d'alcool laurique (6 moles), mais l'inhibiteur de polymérisation est le monométhyléther de l'hydroquinone (250 ppm par rapport aux réactifs). On obtient après 3 heures 30 min de réaction une conversion de 98 %.

Puis le milieu réactionnel est étêté sous vide en continu à 155-165° C sous 70 à 130 mmHg, dans un appareillage muni d'une agitation, d'un thermomètre, d'un plongeant pour le bullage d'air, d'une tête de Claisen et d'un réfrigérant.

La masse étêtée est ensuite traitée dans un ballon de 4 l muni d'une agitation, d'un thermomètre et d'un réfrigérant de droit : la température masse est portée à 65° C, on introduit 2,7 g de soude 50 % et 1,5 g de noir Norit D 10, la température est maintenue à 65° C durant 0 h 20 min, puis on alluvionne avec 3 g de sciure de bois, et on filtre sur précouche de clarcel DIC.

On obtient 1532 g de méthacrylate de lauryle contenant 50 ppm de monométhyléther de l'hydroquinone.

## Revendications

1) Procédé de transestérification du méthacrylate de méthyle par un alcool lourd en présence de lithium comme catalyseur, caractérisé en ce que la quantité de lithium utilisée est comprise entre 6 et 30 ppm (exprimée en lithium) d'hydroxyde ou de carbonate de lithium, que le composé de lithium utilisé est ajouté progressivement au milieu réactionnel et que la réaction est effectuée en présence d'un azéotropeur donnant, avec le méthanol, un azéotrope bouillant à température inférieure à environ 60° C.

2) Procédé selon la revendication 1, caractérisé en ce que le composé azéotrope est l'hexane.

3) Procédé selon l'une des revendications 1 et 2, caractérisé en ce qu'on utilise également comme catalyseur un éther couronne et/ou un cryptant à raison de 0,01 à 2 moles par mole de catalyseur.

## Patentansprüche

1. Verfahren zur Transesterifizierung von Methylmethacrylat mit einem schweren Alkohol in Gegenwart von Lithium als Katalysator, dadurch gekennzeichnet, daß die verwandte Lithiummenge zwischen 6 und 30 ppm (ausgedrückt als Lithium) Lithiumhydroxid oder -carbonat liegt, die verwandte Lithiumverbindung dem Reaktionsmedium progressiv zugesetzt wird und die Reaktion in Gegenwart eines Azeotropbildners, der mit Methanol ein bei einer Temperatur unterhalb von etwa 60°C siedendes Azeotrop ergibt, durchgeführt wird.

2. Verfahren nach Anspruch 1, dadurch gekennzeichnet, daß die azeotrope Verbindung Hexan ist.

3. Verfahren nach einem der Ansprüche 1 und 2, dadurch gekennzeichnet, daß man als Katalysator weiterhin einen Kronenether und/oder einen Kryptanden in einem Verhältnis von 0,01 bis 2 mol pro Mol Katalysator verwendet.

## Claims

1. A process for the transesterification of methyl methacrylate by a heavy alcohol in the presence of lithium as a catalyst, characterised in that the amount of lithium used is between 6 and 30 ppm (expressed as lithium) of lithium hydroxide or carbonate, that the lithium used is progressively added to the reaction medium and that the reaction is carried out in the presence of an azeotroping agent which with methanol gives an azeotrope that boils at a temperature of lower than about 60°C.

2. A process according to claim 1 characterised in that the azeotroping compound is hexane.

3. A process according to one of claims 1 and 2 characterised by also using as the catalyst a crown ether and/or a crypting agent in a proportion of from 0.01 to 2 moles per mole of catalyst.